# EUROPEAN PATENT APPLICATION

(11) **EP 4 071 251 A1**
(43) Date of publication of application: **12.10.2022**
(21) Application number: 22165376.9
(22) Date of filing: 30.03.2022
(51) Int. Cl.: C12Q 1/00, C12Q 1/54, A61B 5/145, A61B 5/1486, A61B 5/00, G01N 27/30

(54) **HEXAMETHYLDISILOXANE MEMBRANES FOR ANALYTE SENSORS**

(30) Priority: 09.04.2021 US 202117226412
(71) Applicant: Medtronic MiniMed, Inc., Northridge, CA 91325-1219 (US)
(72) Inventor: Tsang, Melissa, Sherman Oaks (US); Missaghi, Michael N., Edina (US); Miller, Anna, Woodland Hills (US); Lin, Chi-En, Encino (US); Askarinya, Mohsen, Chandler (US); Srinivasan, Akhil, Woodland Hills (US); Abraham, Nicole, Ojai (US)
(74) Representative: Zimmermann & Partner Patentanwälte mbB

(57) **Abstract**

Embodiments of the invention provide methods and materials for making analyte sensors having a plurality of layered elements such as amperometric glucose sensors that are used by diabetic individuals to monitor blood sugar concentrations. Embodiments of the invention utilize plasma deposition technologies to form thin films of hexamethyldisiloxane useful in such sensors. Sensors that incorporate the thin film compositions formed by these processes exhibit a number of desirable characteristics.

## Description

### BACKGROUND OF THE INVENTION

### 1. Field of the Invention

This invention relates to biosensors such as analyte sensors used in the management of diabetes and in particular methods and materials used to make such sensors.

### 2. Description of Related Art

There are many types of biosensors used to detect wide variety of analytes. Perhaps the most studied type of biosensor is the amperometric glucose sensor, an apparatus commonly used to monitor glucose levels in individuals with diabetes.

A typical glucose sensor works according to the following chemical reactions:

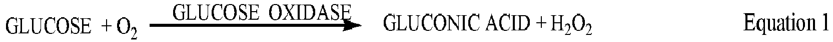

H₂O₂ → O₂ + 2H⁺ + 2e⁻ Equation 2

The glucose oxidase ("GOx") is used to catalyze the reaction between glucose and oxygen to yield gluconic acid and hydrogen peroxide as shown in equation 1. The H₂O₂ reacts electrochemically as shown in equation 2, and the current is measured by a potentiostat. The stoichiometry of certain sensor reactions can provide challenges to developing *in vivo* sensors. For example, for optimal glucose oxidase-based sensor performance, sensor signal output should be determined only by the analyte of interest (glucose), and not by any co-substrates (O₂) or kinetically controlled parameters such as diffusion. If oxygen and glucose are present in equimolar concentrations, then the H₂O₂ is stoichiometrically related to the amount of glucose that reacts at the enzyme; and the associated current that generates the sensor signal is proportional to the amount of glucose that reacts with the enzyme. If, however, there is insufficient oxygen for all of the glucose to react with the enzyme, then the current will be proportional to the oxygen concentration, not the glucose concentration. Consequently, for the sensor to provide a signal that depends solely on the concentrations of the analyte of interest, glucose must be the limiting reagent, i.e., the O₂ concentration must be in excess for all potential glucose concentrations. A problem with using such glucose sensors *in vivo,* however, is that the oxygen concentration where the sensor is implanted *in vivo* is low relative to glucose, a phenomenon which can compromise the accuracy of sensor readings.

Certain sensor designs address stoichiometric issues such as the oxygen deficit problem by using a series of layered materials selected to have specific function properties, for example an ability to selectively modulate the diffusion of analytes and other pertinent compounds therethrough. Problems associated with such designs can include, for example, sensor layers delaminating and/or degrading over time in a manner that can limit the functional lifetime of the sensor. Methods and materials designed to address such challenges in this technology are desirable.

### SUMMARY OF THE INVENTION

Embodiments of the invention include plasma processes for making hexamethyldisiloxane (HMDSO) layers in analyte sensors. The plasma processes disclosed herein have a number of advantages over conventional wet chemistry processes used to form conventional sensor layers, including reducing and/or eliminating the use of certain hazardous compounds, thereby reducing toxic wastes that can result from such processes. Embodiments of the invention also include analyte sensors designed to include hexamethyldisiloxane layers/compositions formed from these processes, compositions that exhibit a combination of desirable material properties including relatively thin and highly uniform structural profiles.

Illustrative embodiments of the invention include methods of making an analyte sensor apparatus from a plurality of layered materials including one or more layers comprising hexamethyldisiloxane formed from a plasma vapor deposition process. Embodiments of the invention include, for example, methods of making an analyte sensor apparatus comprising: providing a base layer; forming a conductive layer over the base layer, where this conductive layer includes a working electrode; forming an analyte sensing layer over the conductive layer, where this analyte sensing layer includes a composition that can alter the electrical current at the working electrode in the conductive layer in the presence of an analyte (e.g. glucose or 3-hydroxybutyrate); and then forming an analyte modulating layer over the analyte sensing layer. In such embodiments, the analyte modulating layer is formed to inhibit the diffusion of glucose, 3-hydroxybutyrate and/or nicotinamide adenine dinucleotide (NAD) therethrough; and comprises a layer of HDMSO formed over the analyte sensing layer using a plasma vapor deposition process. In illustrative embodiments of the invention, the plasma vapor deposition process used to generate the layer of HDMSO comprises a methodological step that includes: a duration of between 40-400 seconds; a pressure of between 50 and 200 mTorr; a RF power of between 140-210 W; a hexamethyldisiloxane flow rate of between 10 and 20 sccm; and/or a N₂O flow rate of between 10 and 50 sccm. Certain embodiments include performing a pretreatment step on a layer, where this pretreatment step comprises exposure to a gas plasma.

A related embodiment of the invention is an analyte sensor apparatus comprising a constellation of elements including a base layer; a conductive layer disposed over the base layer, where this conductive layer includes a working electrode; an analyte sensing layer disposed over the conductive layer, where this analyte sensing layer includes a composition that can alter the electrical current at the working electrode in the conductive layer in the presence of an analyte (an enzyme selected from the group consisting of glucose oxidase and 3-hydroxybutyrate dehydrogenase); and also a layer of HDMSO disposed over the analyte sensing layer (e.g. an analyte modulating layer having the material and functional properties that result from plasma deposited HDMSO). In certain embodiments of the invention, this layer of HDMSO modulates the diffusion of glucose, 3-hydroxybutyrate or nicotinamide adenine dinucleotide therethrough. In some embodiments of the invention, the analyte sensor apparatus senses glucose. In certain embodiments of the invention, the analyte sensor apparatus senses 3-hydroxybutyrate.

Other embodiments of the invention include methods of sensing analytes within the body of a mammal using an amperometric sensor that comprises a plurality of layered materials including a layer formed from plasma vapor deposited hexamethyldisiloxane as disclosed herein and which is disposed in the sensor to modulate the diffusion of glucose, 3-hydroxybutyrate and/or nicotinamide adenine dinucleotide (NAD) therethrough. Typically, the methods comprise implanting the analyte sensor into the mammal, sensing an alteration in current at a sensor electrode in the presence of the analyte; and correlating the alteration in current with the presence and/or concentration of the analyte. In illustrative embodiments, the sensor is a glucose sensor used by a diabetic patient.

Further disclosed herein are methods and materials for making analyte sensors having a plurality of layered elements such as amperometric glucose sensors that are used by diabetic individuals to monitor blood sugar concentrations, wherein embodiments utilize plasma deposition technologies to form thin films of hexamethyldisiloxane useful in such sensors, wherein sensors incorporate the thin film compositions formed by these processes exhibit a number of desirable characteristics.

Other objects, features and advantages of the present invention will become apparent to those skilled in the art from the following detailed description. It is to be understood, however, that the detailed description and specific examples, while indicating some embodiments of the present invention are given by way of illustration and not limitation. Many changes and modifications within the scope of the present invention may be made without departing from the spirit thereof, and the invention includes all such modifications.

### BRIEF DESCRIPTION OF THE FIGURES

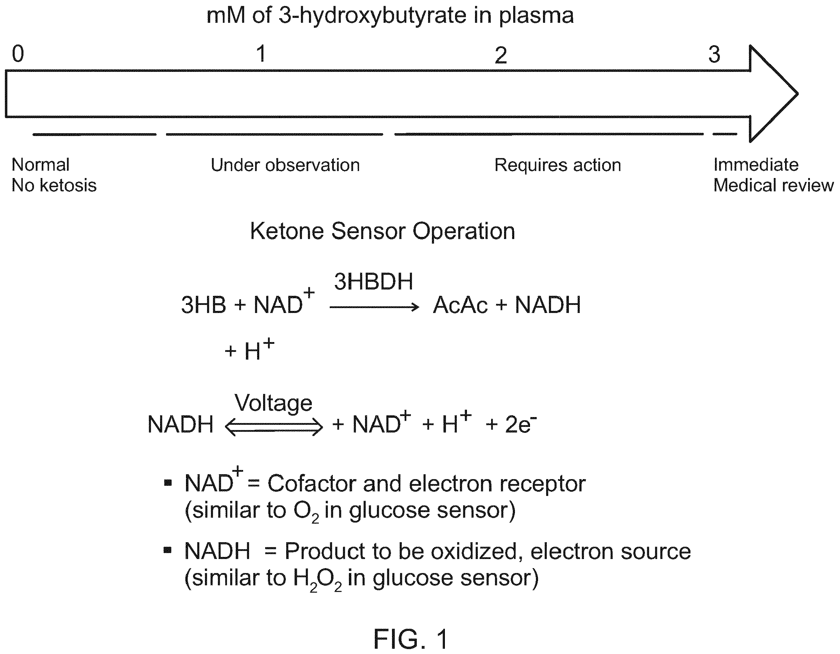

**Figure 1** provides schematics showing the significance of increasing concentrations of 3-hydroxybutyrate ("3HB") in plasma (top panel); and reactions that occur during ketone sensor operation (bottom panel).

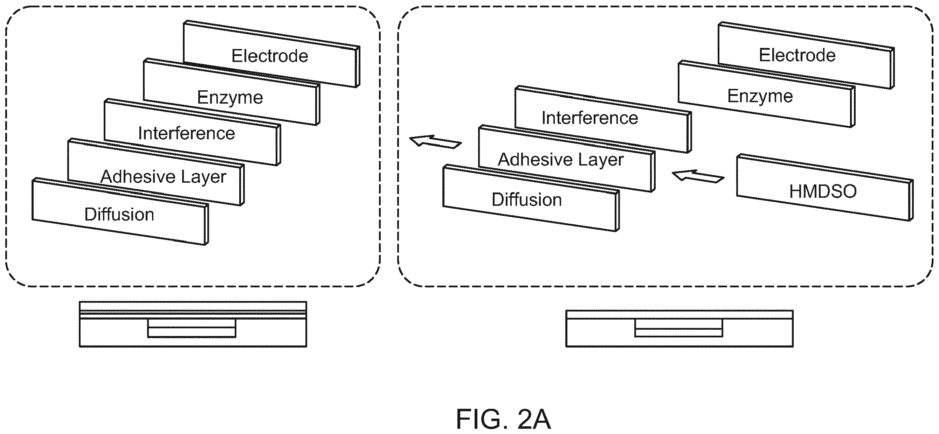

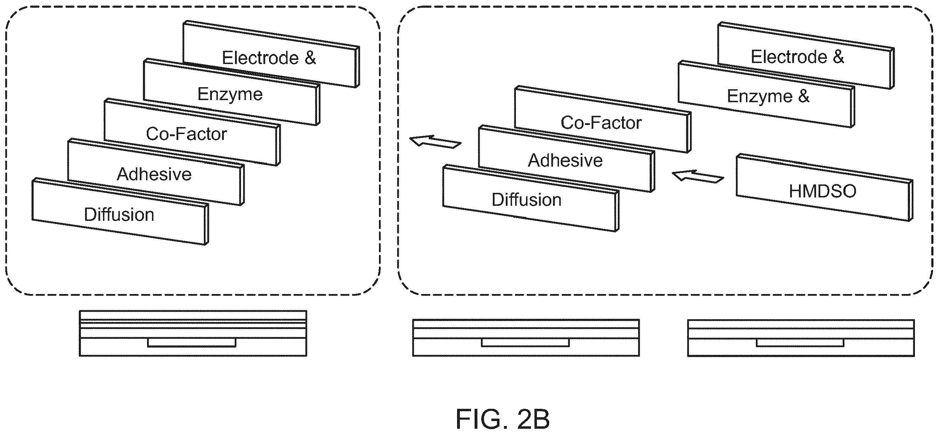

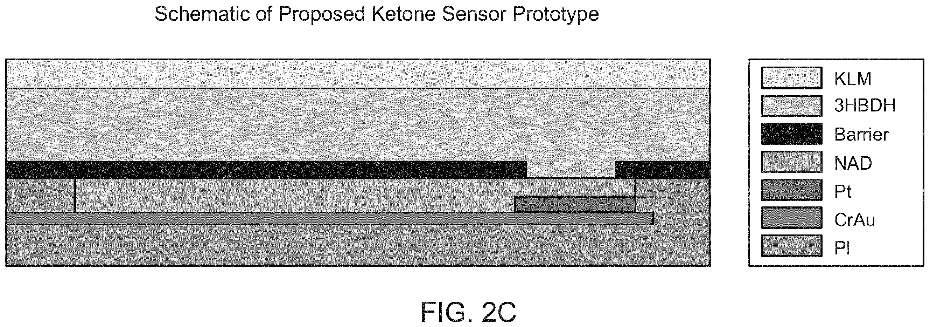

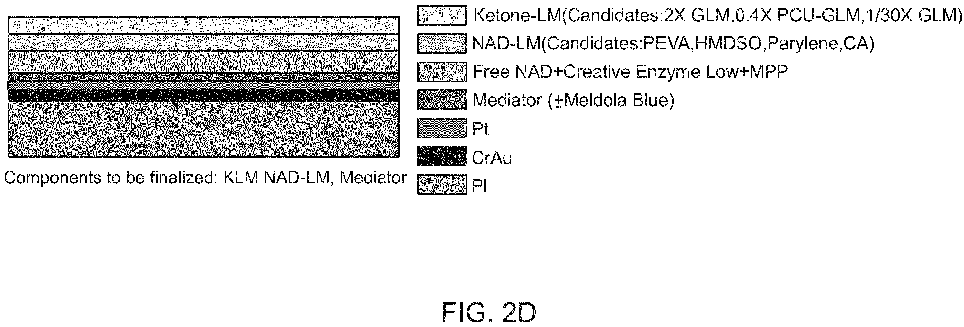

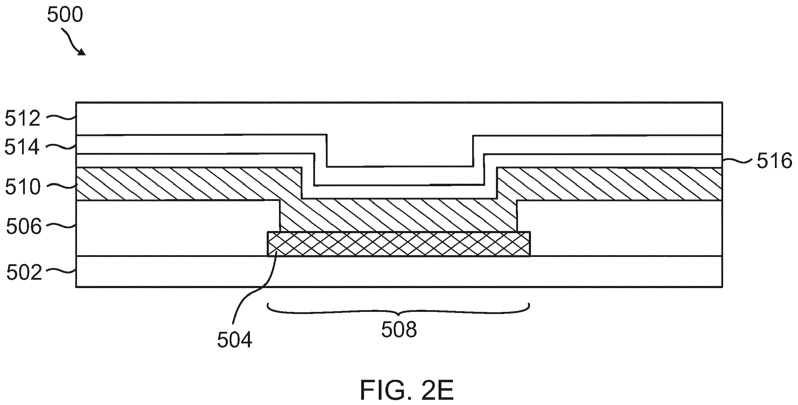

**Figures 2A-2E** provide schematics showing sensor designs comprising views of amperometric analyte sensors formed from a plurality of planar layered elements, for example one or more layers having HDMSO. **Figure 2A** and **Figure 2B** provide schematics showing sensor designs comprising expanded (top panels) and cross sectional (bottom panels) views of amperometric analyte sensors formed from a plurality of planar layered elements, for example one or more layers comprising HDMSO. **Figure 2A** provides a schematic showing material layers found in a first glucose sensor embodiment (left panel); and schematic showing material layers including HDMSO (right panel). **Figure 2B** provides a schematic showing material layers found in a first ketone sensor embodiment (left panel); and schematic showing material layers in a second embodiment including HDMSO (right panel). **Figure 2C** provides a schematic showing sensor embodiments including a barrier layer. **Figure 2D** provides a schematic showing sensor embodiments including, for example, ketone and NAD limiting membranes. **Figure 2E** provides a schematic of a sensor design, for example an amperometric analyte sensor formed from a plurality of planar layered elements in which one or more layers comprise HDMSO.

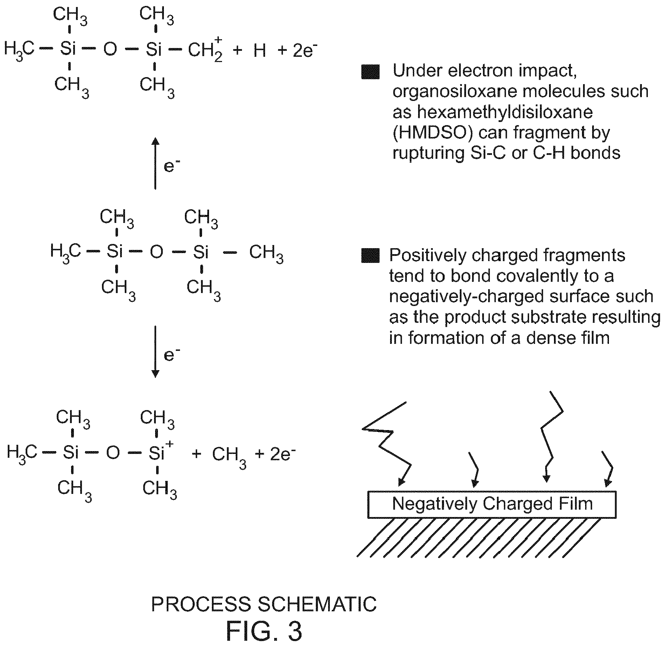

**Figure 3** provides a diagram illustrating a schematic of a generalized/illustrative plasma AP process on a sensor plate.

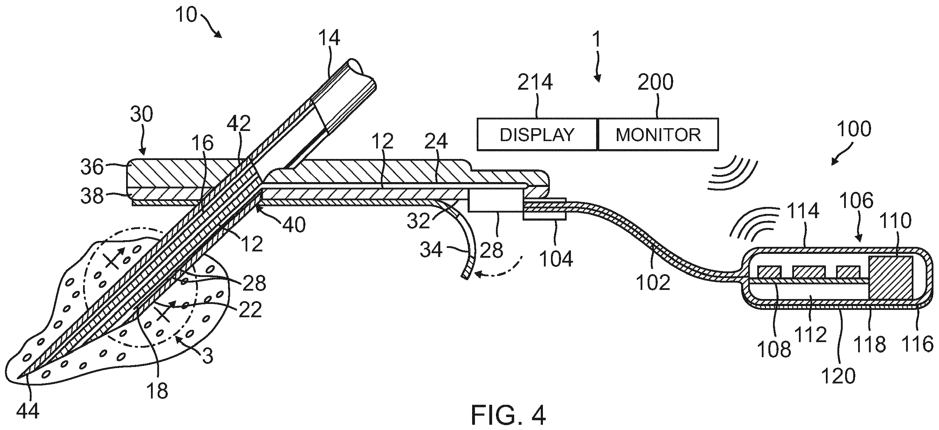

**Figure 4** provides a perspective view illustrating a subcutaneous sensor insertion set, a telemetered characteristic monitor transmitter device, and a data receiving device embodying features of the invention.

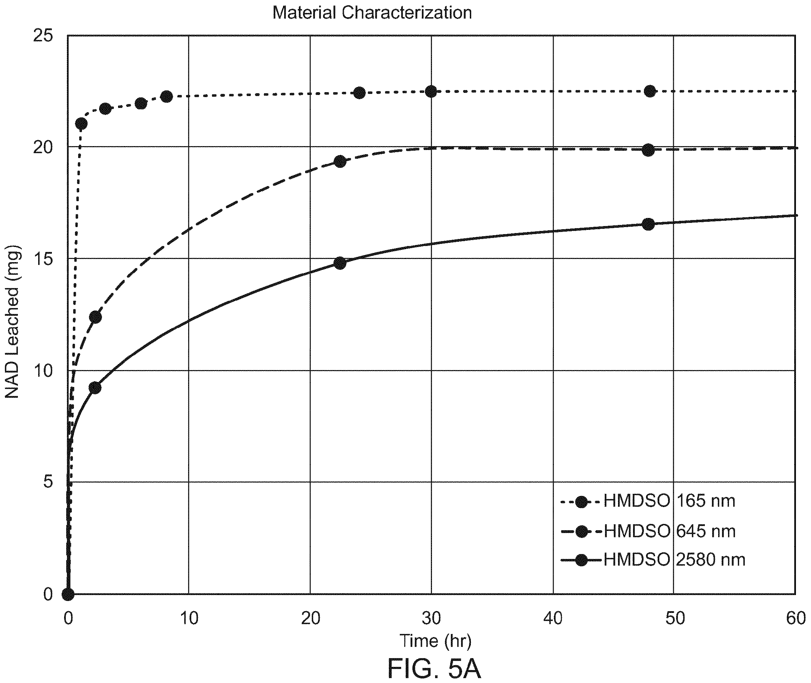

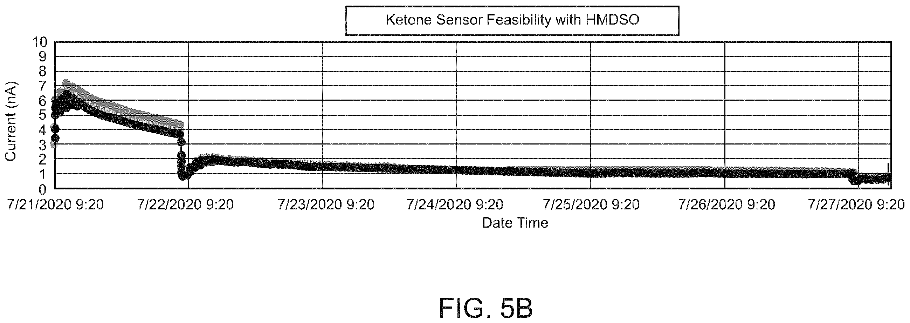

**Figures 5A-5B** provide data from comparative studies of ketone sensor structures that include a layer of HDMSO. **Figure 5A** provides a graph of data showing how layers of the hexamethyldisiloxane compositions disclosed herein having varying thicknesses can be used to selectively modulate/inhibit the diffusion of nicotinamide adenine dinucleotide (NAD+/NADH, "NAD") therethrough; and in this way can, for example, be used to inhibit NAD leaching from an underlying sensor layer comprising this compound. From studies of sensor structures that include HMDSO as a barrier layer designed to inhibit NAD leaching, we see in this graphed data that HMDSO membranes are able to limit NAD leaching. Moreover, with increasing HDMSO film thicknesses we see associated reduced leaching as a result. With a test sensor structure having a layer of HDMSO of 165nm thickness, NAD leaching is observed within the first hour and with thicker layers (645nm and 2580nm) we see that NAD takes significant additional time to leach out. **Figure 5B** provides data from results of the comparative 1-day-long *in vitro* tests showing that sensors comprising an analyte modulating layer formed from HDMSO. These sensors exhibit a greater Isig stability on day 1 as compared to those having an analyte modulating layer formed from a conventional polyurethane composition ("PCU-GLM").

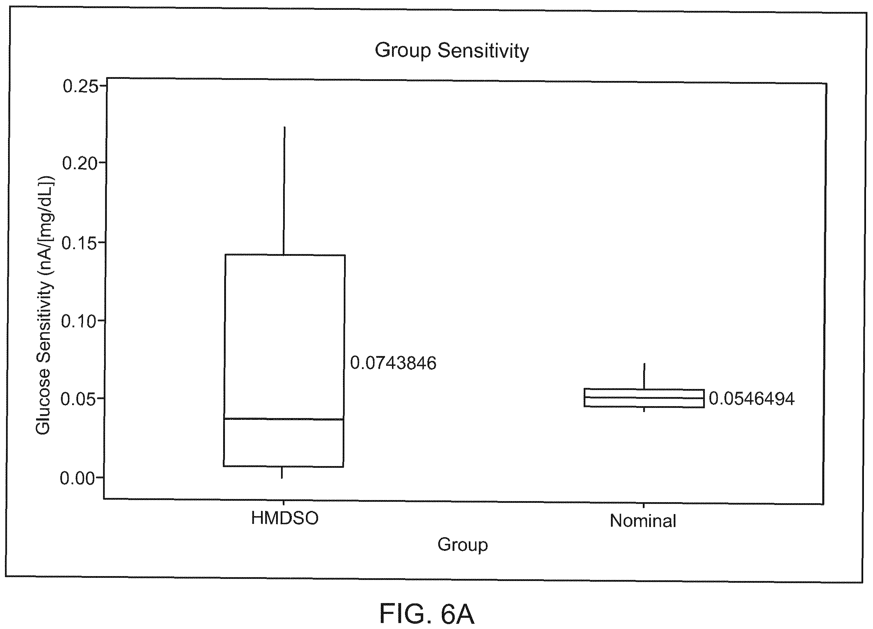

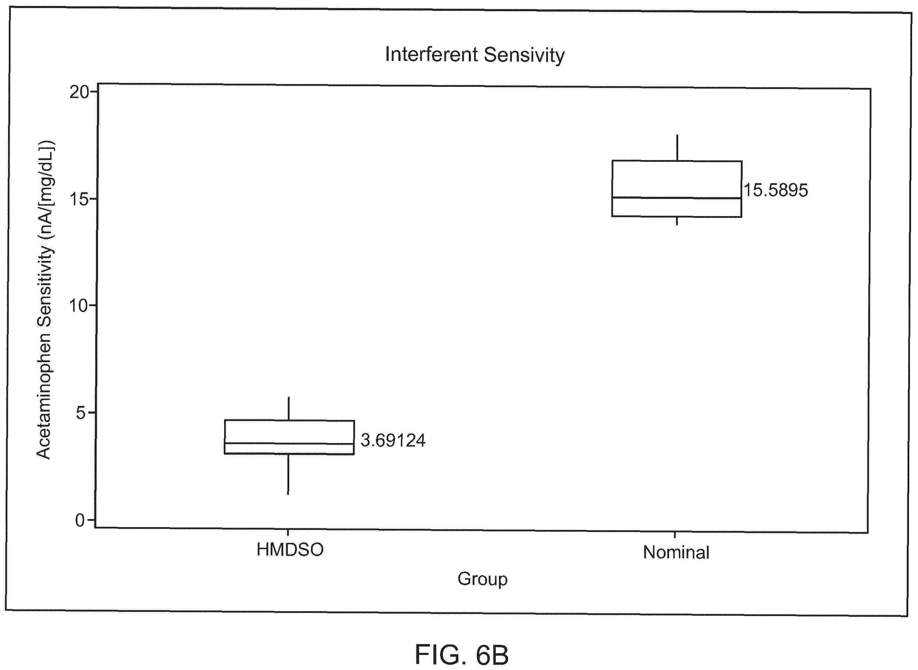

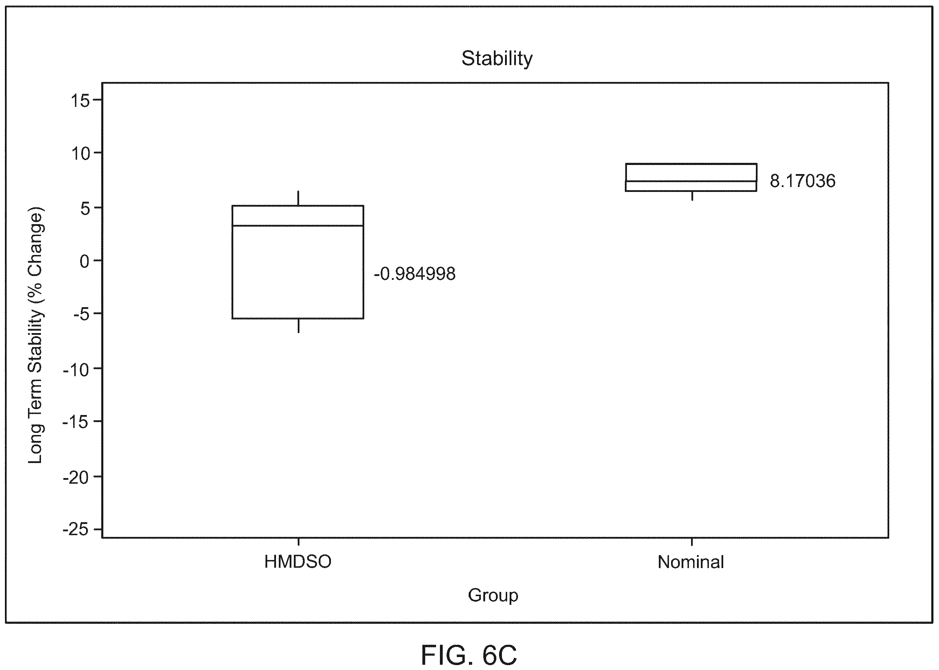

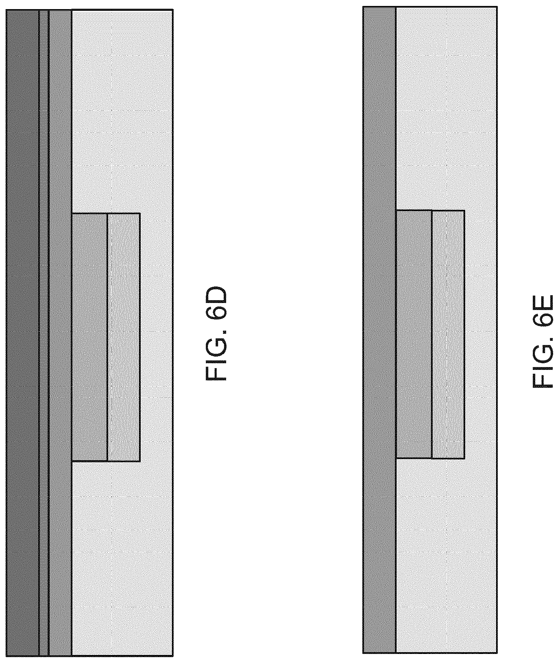

**Figures 6A-6E** provide data and schematics of comparative studies of glucose sensor structures formed to include a layer of HMDSO as a diffusion limiting membrane as compared to sensor structures formed to include a layer of a conventional polyurethane composition as a diffusion limiting membrane ("nominal"). **Figure 6A** shows comparative studies of glucose sensitivity in sensor embodiments comprising a HDMSO analyte modulating layer. **Figure 6B** shows comparative studies of interferent sensitivity in sensor embodiments comprising a HDMSO analyte modulating layer. **Figure 6C** shows comparative studies of sensor stability in sensor embodiments comprising a HDMSO analyte modulating layer. **Figure 6D** provides a cross sectional view of a nominal sensor structure, while **Figure 6E** provides a cross sectional view of a HDMSO sensor structure.

### DETAILED DESCRIPTION OF THE EMBODIMENTS

Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. Many of the techniques and procedures described or referenced herein are well understood and commonly employed using conventional methodology by those skilled in the art. All publications mentioned herein are incorporated herein by reference to disclose and describe the methods and/or materials in connection with which the publications are cited (see, e.g., U. S. Patent No. 7,906,217 and United States Patent Application No. 20070202612). Publications cited herein are cited for their disclosure prior to the filing date of the present application. Nothing here is to be construed as an admission that the inventors are not entitled to antedate the publications by virtue of an earlier priority date or prior date of invention. Further the actual publication dates may be different from those shown and require independent verification.

It must be noted that as used herein and in the appended claims, the singular forms "a", "and", and "the" include plural referents unless the context clearly dictates otherwise. All numbers recited in the specification and associated claims that refer to values that can be numerically characterized with a value other than a whole number (e.g., "60 nanometers") are understood to be modified by the term "about".

The term "sensor," as used herein, is a broad term and is used in its ordinary sense, including, without limitation, elements of an analyte-monitoring device that detect an analyte. In one embodiment, the sensor includes an electrochemical cell that has a working electrode, a reference electrode, and a counter electrode (e.g., a plurality of each of these electrodes) passing through and secured within the sensor body forming an electrochemically reactive surface at one location on the body, an electronic connection at another location on the body, and a membrane system affixed to the body and covering the electrochemically reactive surface. During general operation of the sensor, a biological sample (for example, blood or interstitial fluid), or a portion thereof, contacts (directly or after passage through one or more membranes or domains) an enzyme (for example, glucose oxidase); the reaction of the biological sample (or portion thereof) results in the formation of reaction products that allow a determination of the analyte level in the biological sample.

Embodiments of the invention disclosed herein provide sensors of the type used, for example, in subcutaneous or transcutaneous monitoring of blood glucose levels in a diabetic patient. A variety of implantable, electrochemical biosensors have been developed for the treatment of diabetes and other life-threatening diseases. Many existing sensor designs use some form of immobilized enzyme to achieve their bio-specificity. Embodiments of the invention described herein can be adapted and implemented with a wide variety of known electrochemical sensors, including for example, U.S. Patent Application No. 20050115832, U.S. Pat. Nos. 6,001,067, 6,702,857, 6,212,416, 6,119,028, 6,400,974, 6,595,919, 6,141,573, 6,122,536, 6,512,939 5,605,152, 4,431,004, 4,703,756, 6,514,718, 5,985,129, 5,390,691, 5,391, 250, 5,482,473, 5,299,571, 5,568,806, 5,494,562, 6,120,676, 6,542,765, 8,703,354 as well as PCT International Publication Numbers WO 01/58348, WO 04/021877, WO 03/034902, WO 03/035117, WO 03/035891, WO 03/023388, WO 03/022128, WO 03/022352, WO 03/023708, WO 03/036255, WO03/036310 WO 08/042625, and WO 03/074107, and European Patent Application EP 1153571, the contents of each of which are incorporated herein by reference.

A wide variety of sensors and sensor elements are known in the art including amperometric sensors used to detect and/or measure biological analytes such as glucose. Many glucose sensors are based on an oxygen (Clark-type) amperometric transducer (see, e.g. Yang et al., Electroanalysis 1997, 9, No. 16: 1252-1256; Clark et al., Ann. N.Y. Acad. Sci. 1962, 102, 29; Updike et al., Nature 1967, 214,986; and Wilkins et al., Med. Engin. Physics, 1996, 18, 273.3-51). Electrochemical glucose sensors that utilize the chemical reaction between glucose and glucose oxidase to generate a measurable signal typically include polymeric compositions that modulate the diffusion of analytes including glucose in order to overcome stoichiometric issues such as what is known in the art as the "oxygen deficit problem". Specifically, because glucose oxidase based sensors require both oxygen (O₂) as well as glucose to generate a signal, the presence of an excess of oxygen relative to glucose, is necessary for the operation of a glucose oxidase based glucose sensor. However, because the concentration of oxygen in subcutaneous tissue is much less than that of glucose, oxygen can be the limiting reactant in the reaction between glucose, oxygen, and glucose oxidase in a sensor, a situation which compromises the sensor's ability to produce a signal that is strictly dependent on the concentration of glucose. The modification and/or substitution of layered materials in a sensor can be problematical in that such modifications can result in unpredictable alterations in the crucial permselective properties of these layers. For example, because the properties of a material can influence the rate at which compounds diffuse through that material to the site of a measurable chemical reaction, the material properties of an analyte modulating layer used in electrochemical glucose sensors that utilize the chemical reaction between glucose and glucose oxidase to generate a measurable signal, should not for example, favor the diffusion of glucose over oxygen in a manner that contributes to the oxygen deficit problem. In this context, the plasma deposited hexamethyldisiloxane layers that are disclosed herein exhibit functional features including diffusion profiles that make them useful in layered sensor structures designed to address stoichiometric issues such as the oxygen deficit problem that is observed in amperometric glucose sensors. These materials can be used to make sensors having a number of desirable properties, including an extended shelf life as well as enhanced performance profiles.

As discussed in detail below, embodiments of the invention disclosed herein provide sensor elements having enhanced material properties and sensor systems (e.g., those comprising a sensor and associated electronic components such as a monitor, a processor and the like) constructed to include such elements. The disclosure further provides methods for making and using such sensors. While some embodiments of the invention pertain to glucose and ketone sensors, a variety of the processes and materials disclosed herein (e.g., glucose, 3-hydroxybutyrate or NAD modulating layers formed from HDMSO using plasma deposition processes) can be adapted for use with any one of the wide variety of analyte sensors known in the art. Such sensors of the invention exhibit a surprising degree of flexibility and versatility, characteristics which allow a wide variety of sensor configurations to be designed to examine a wide variety of analytes. The term "analyte" as used herein is a broad term and is used in its ordinary sense, including, without limitation, to refer to a substance or chemical constituent in a fluid such as a biological fluid (for example, blood, interstitial fluid, cerebral spinal fluid, lymph fluid or urine) that can be analyzed. Analytes can include naturally occurring substances, artificial substances, metabolites, and/or reaction products. In illustrative embodiments, the analyte is glucose and/or 3-hydroxybutyrate.

Specific aspects of embodiments of the invention are discussed in detail in the following sections.

### TYPICAL PROCESSES, ELEMENTS, AND ANALYTE SENSORS OF THE INVENTION

As discussed in detail below, embodiments of the invention relate to methods for making and using an electrochemical sensor that exhibits a novel constellation of elements including a HDMSO layer (e.g., one that functions as a layer that modulates the diffusion of glucose, 3-hydroxybutyrate and/or NAD) having a unique set of features including improved material properties as well as an ease in manufacture. The electrochemical sensors of the invention are designed to measure a concentration of an analyte of interest (e.g., glucose or 3-hydroxybutyrate) or a substance indicative of the concentration or presence of the analyte in fluid. In some embodiments, the sensor is a continuous device, for example a subcutaneous, transdermal, or intravascular device. In some embodiments, the device can analyze a plurality of intermittent blood samples to provide an output signal indicative of the concentration of the analyte of interest. Such sensors comprise one or more HMDSO layers having a constellation of selected material properties, including for example, allowing glucose and/or oxygen and/or 3-hydroxybutyrate and/or nicotinamide adenine dinucleotide and/or oxygen, to permselectively migrate through these layers prior to reacting with a sensing complex (e.g., an enzyme such as glucose oxidase disposed on an electrode). The presence of the analyte can be measured using electrochemical methods and the output of an electrode system can function as a measure of the analyte. Typically, the sensor is of the type that senses a product or reactant of an enzymatic reaction between an analyte and an enzyme in the presence of oxygen as a measure of the analyte *in vivo* or *in vitro* (e.g., hydrogen peroxide generated by glucose oxidase in the presence of glucose).

As noted above, embodiments of the invention relate to layered sensor structures, for example those found in amperometric glucose sensors having a plurality of layered materials including one or more HMDSO materials/layers. Illustrative embodiments of the invention include methods of making an analyte sensor apparatus from a plurality of layered materials including one or more layers comprising hexamethyldisiloxane formed from a plasma vapor deposition process (e.g., a pulse deposition process, a dual plasma vapor deposition process or the like). Embodiments of the invention include, for example, methods of making an analyte sensor apparatus comprising: providing a base layer; forming a conductive layer over the base layer, wherein the conductive layer includes a working electrode; forming an analyte sensing layer over the conductive layer, wherein the analyte sensing layer includes a composition that can alter the electrical current at the working electrode in the conductive layer in the presence of an analyte; and then forming an analyte modulating layer over the analyte sensing layer. In such embodiments, the analyte modulating layer is formed to inhibit the diffusion of glucose, 3-hydroxybutyrate and/or nicotinamide adenine dinucleotide (NAD) therethrough; and further comprises a layer of HDMSO formed over the analyte sensing layer using a plasma vapor deposition process. In illustrative embodiments of the invention, the plasma vapor deposition process used to generate the layer of HDMSO comprises a methodological step that includes: a duration of between 40-400 seconds (e.g. from 40 to 60 seconds, from 40-45 seconds etc.); a pressure of between 50 and 200 mTorr (e.g. from 125 to 150 mTorr); a RF power of between 140-210 W; a hexamethyldisiloxane flow rate of from 10 to 20 sccm; a N₂O flow rate of between 10 and 50 sccm (e.g. about 30 sccm) and/or an Ar flow rate of about 1 sccm. HDMSO layers made in this way exhibit a number of unexpected properties disclosed herein. Certain embodiments include performing a pretreatment step on a layer, wherein the pretreatment step comprises exposure to a gas plasma. In certain methodological embodiments of the invention, the thickness and/or architecture (e.g., the presence of a port) of a layer of HDMSO is selected so that the layer inhibits NAD leaching from a layer in the sensor and out of the sensor into the external environment by at least 25% or at least 50% over a period of 24 hours as compared to a control sensor having no HDMSO barrier layer.

In typical embodiments of the invention, the analyte sensing layer is formed to comprise an enzyme selected from the group consisting of glucose oxidase and 3-hydroxybutyrate dehydrogenase (see, e.g., U.S. Patent Publication Nos. 20180346886 and 20180291354). In certain embodiments of the invention, the analyte modulating layer is formed to be between 100nm and 1000nm in thickness. This thickness range has been discovered to be useful to modulate the diffusion of NAD in ketone sensors (see, Figure 5). In certain embodiments of the invention, the methods further comprise forming the sensor to include one or more additional layers such as: an adhesion promoting layer; an interference rejection layer; a layer comprising nicotinamide adenine dinucleotide; a barrier layer that inhibits the diffusion of nicotinamide adenine dinucleotide therethrough; and/or a protein layer. Optionally one or more of these layers comprises HDMSO. In certain embodiments of the invention, a barrier layer is formed to be between 100nm and 1000nm in thickness; and/or the barrier layer is formed to comprise a port.

A related embodiment of the invention is an analyte sensor apparatus comprising a constellation of elements including a base layer; a conductive layer disposed over the base layer, wherein the conductive layer includes a working electrode; an analyte sensing layer disposed over the conductive layer, wherein the analyte sensing layer includes a composition that can alter the electrical current at the working electrode in the conductive layer in the presence of an analyte (an enzyme selected from the group consisting of glucose oxidase and 3-hydroxybutyrate dehydrogenase); a layer of HDMSO disposed over the analyte sensing layer (e.g. an analyte modulating layer). In such embodiments of the invention, this layer of HDMSO modulates the diffusion of glucose, 3-hydroxybutyrate or nicotinamide adenine dinucleotide therethrough. Typically, the HDMSO layer is selected to be of a certain thickness, for example, between 100nm and 1000nm in thickness, or not more than 500nm or 1000nm in thickness. In some embodiments of the invention, the analyte sensor apparatus senses glucose. In certain embodiments of the invention, the analyte sensor apparatus senses 3-hydroxybutyrate.

In certain embodiments of the invention, the analyte sensor apparatus further comprises one or more additional layers (optionally comprising HDMSO) in the sensor comprising: an adhesion promoting layer; a layer comprising nicotinamide adenine dinucleotide; a barrier layer that inhibits the diffusion of nicotinamide adenine dinucleotide therethrough; a high-density amine layer; or a protein layer. In some embodiments of the invention, a layer in the sensor comprises a diisocyanate; a hydrophilic polymer comprising a hydrophilic diol or hydrophilic diamine; a siloxane having an amino, hydroxyl or carboxylic acid functional group at a terminus; and/or a polycarbonate diol.

In some embodiments, the HMDSO layer is disposed over another layer such as a protein layer that is disposed over the analyte sensing layer, for example a protein layer comprising bovine serum albumin (BSA) or human serum albumin (HSA). In typical embodiments, the protein constituent in this layer comprises an albumin such as human serum albumin. The HSA concentration may vary between about 0.5%-30% (w/v). Typically, the HSA concentration is about 1-10% w/v, and most typically is about 5% w/v. In alternative embodiments of the invention, collagen or BSA or other structural proteins used in these contexts can be used instead of or in addition to HSA. Embodiments on the invention include further layers disposed over a HMDSO layer. In certain embodiments of the invention, an analyte modulating layer comprises HMDSO. In other embodiments of the invention, the analyte modulating layer comprises a linear polyurethane/polyurea polymer; and another layer of the sensor comprises HMDSO. Conventionally, analyte modulating layers are formed from other compounds such as a mixture comprising: a diisocyanate compound (typically about 50 mol% of the reactants in the mixture); at least one hydrophilic diol or hydrophilic diamine compound (typically about 17 to 45 mol% of the reactants in the mixture); and a siloxane compound. Optionally the polyurethane/polyurea polymer comprises 45-55 mol% (e.g., 50 mol%) of a diisocyanate (e.g., 4,4'-diisocyanate), 10-20 (e.g. 12.5 mol%) mol% of a siloxane (e.g. polymethylhydrosiloxane, trimethylsilyl terminated), and 30-45 mol% (e.g. 37.5 mol%) of a hydrophilic diol or hydrophilic diamine compound (e.g. polypropylene glycol diamine having an average molecular weight of 600 Daltons, Jeffamine 600). In certain embodiments of the analyte modulating layer a first polyurethane/polyurea polymer is blended with a second polymer formed from a mixture comprising: 5-45 weight % of a 2-(dimethylamino)ethyl methacrylate compound; 15-55 weight % of a methyl methacrylate compound; 15-55 weight % of a polydimethyl siloxane monomethacryloxypropyl compound; 5-35 weight % of a poly(ethylene oxide) methyl ether methacrylate compound; and 1-20 weight % 2-hydroxyethyl methacrylate, with the first polymer and the second polymer blended together at a ratio between 1:1 and 1:20 weight %.

Other embodiments of the invention include methods of sensing analytes within the body of a mammal using an amperometric sensor that comprises a plurality of layered materials including a layer formed from plasma vapor deposited hexamethyldisiloxane as disclosed herein and which is disposed in the sensor to modulate the diffusion of glucose, 3-hydroxybutyrate and/or nicotinamide adenine dinucleotide (NAD) therethrough. Typically, the methods comprise implanting the analyte sensor into the mammal, sensing an alteration in current at a sensor electrode in the presence of the analyte; and correlating the alteration in current with the presence and/or concentration of the analyte. In illustrative embodiments, the sensor is an analyte sensor used by a diabetic patient.

### Typical Analyte Sensor Constituents Used in Embodiments of the Invention

Figures 2 and 4 provide illustrations of various sensor and sensor system embodiments of the invention. The following disclosure provides examples of typical elements/constituents used in sensor embodiments of the invention which can include a HDMSO material. While these elements can be described as discreet units (e.g. layers), those of skill in the art understand that sensors can be designed to contain elements having a combination of some or all of the material properties and/or functions of the elements/constituents discussed below (e.g. an element that serves both as a supporting base constituent and/or a conductive constituent and/or a matrix for the analyte sensing constituent and which further functions as an electrode in the sensor). Those in the art understand that these thin film analyte sensors can be adapted for use in a number of sensor systems such as those described below.

### Base Constituent

Sensors of the invention typically include a base constituent (see, e.g., element 502 in Figure 2E). The term "base constituent" is used herein according to art accepted terminology and refers to the constituent in the apparatus that typically provides a supporting matrix for the plurality of constituents that are stacked on top of one another and comprise the functioning sensor. This base constituent can be made of a wide variety of materials having desirable qualities such as the constellation of features disclosed herein as well as dielectric properties, water impermeability and hermeticity. Some illustrative base materials include metallic, and/or ceramic and/or polymeric substrates or the like.

### Conductive Constituent

The electrochemical sensors of the invention typically include a conductive constituent disposed upon the base constituent that includes at least one electrode for contacting an analyte or its byproduct (e.g., oxygen and/or hydrogen peroxide) to be assayed (see, e.g., element 504 in Figure 2E). The term "conductive constituent" is used herein according to art accepted terminology and refers to electrically conductive sensor elements such as a plurality of electrically conductive members disposed on the base layer in an array (e.g., so as to form a microarray electrode) and which are capable of measuring a detectable signal and conducting this to a detection apparatus. An illustrative example of this is a conductive constituent that forms a working electrode that can measure an increase or decrease in current in response to exposure to a stimuli such as the change in the concentration of an analyte or its byproduct as compared to a reference electrode that does not experience the change in the concentration of the analyte, a coreactant (e.g. oxygen) used when the analyte interacts with a composition (e.g. the enzyme glucose oxidase) present in analyte sensing constituent 510 or a reaction product of this interaction (e.g. hydrogen peroxide). Illustrative examples of such elements include electrodes which are capable of producing variable detectable signals in the presence of variable concentrations of molecules such as hydrogen peroxide or oxygen.

In addition to the working electrode, the analyte sensors of the invention typically include a reference electrode or a combined reference and counter electrode (also termed a quasi-reference electrode or a counter/reference electrode). If the sensor does not have a counter/reference electrode then it may include a separate counter electrode, which may be made from the same or different materials as the working electrode. Typical sensors of the present invention have one or more working electrodes and one or more counter, reference, and/or counter/reference electrodes. One embodiment of the sensor of the present invention has two, three or four or more working electrodes. These working electrodes in the sensor may be integrally connected or they may be kept separate. Optionally, the electrodes can be disposed on a single surface or side of the sensor structure. Alternatively, the electrodes can be disposed on a multiple surfaces or sides of the sensor structure (and can for example be connected by vias through the sensor material(s) to the surfaces on which the electrodes are disposed). In certain embodiments of the invention, the reactive surfaces of the electrodes are of different relative areas/sizes, for example a IX reference electrode, a 2.6X working electrode and a 3.6X counter electrode.

### Interference Rejection Constituent

The electrochemical sensors of the invention optionally include an interference rejection constituent disposed between the surface of the electrode and the environment to be assayed. In particular, certain sensor embodiments rely on the oxidation and/or reduction of hydrogen peroxide generated by enzymatic reactions on the surface of a working electrode at a constant potential applied. Because amperometric detection based on direct oxidation of hydrogen peroxide requires a relatively high oxidation potential, sensors employing this detection scheme may suffer interference from oxidizable species that are present in biological fluids such as ascorbic acid, uric acid and acetaminophen. In this context, the term "interference rejection constituent" is used herein according to art accepted terminology and refers to a coating or membrane in the sensor that functions to inhibit spurious signals generated by such oxidizable species which interfere with the detection of the signal generated by the analyte to be sensed. Certain interference rejection constituents function via size exclusion (e.g., by excluding interfering species of a specific size). Examples of interference rejection constituents include one or more layers or coatings of compounds such as hydrophilic polyurethanes, cellulose acetate (including cellulose acetate incorporating agents such as poly(ethylene glycol), polyethersulfones, polytetra-fluoroethylenes, the perfluoronated ionomer Nafion^{™}, polyphenylenediamine, epoxy and the like.

### Analyte Sensing Constituent

The electrochemical sensors of the invention include an analyte sensing constituent disposed on the electrodes of the sensor (see, e.g., element 510 in Figure 2E). The term "analyte sensing constituent" is used herein according to art accepted terminology and refers to a constituent comprising a material that is capable of recognizing or reacting with an analyte whose presence is to be detected by the analyte sensor apparatus. Typically, this material in the analyte sensing constituent produces a detectable signal after interacting with the analyte to be sensed, typically via the electrodes of the conductive constituent. In this regard, the analyte sensing constituent and the electrodes of the conductive constituent work in combination to produce the electrical signal that is read by an apparatus associated with the analyte sensor. Typically, the analyte sensing constituent comprises an enzyme capable of reacting with and/or producing a molecule whose change in concentration can be measured by measuring the change in the current at an electrode of the conductive constituent (e.g., oxygen and/or hydrogen peroxide), for example the enzyme glucose oxidase. While glucose oxidase based glucose sensors are used as an illustrative example, those of skill in the art understand that many aspects of the invention similarly pertain to other sensors such as 3-hydroxybutyrate dehydrogenase-based ketone sensors. The analyte sensing constituent can coat all or a portion of the various electrodes of the sensor. In this context, the analyte sensing constituent may coat the electrodes to an equivalent degree. Alternatively, the analyte sensing constituent may coat different electrodes to different degrees, with for example the coated surface of the working electrode being larger than the coated surface of the counter and/or reference electrode.

Typical sensor embodiments of this element of the invention utilize an enzyme (e.g., glucose oxidase) that has been combined with a second protein (e.g., albumin) in a fixed ratio (e.g. one that is typically optimized for glucose oxidase stabilizing properties) and then applied on the surface of an electrode to form a thin enzyme constituent. In a typical embodiment, the analyte sensing constituent comprises a GOx and HSA mixture. In a typical embodiment of an analyte sensing constituent having GOx, the GOx reacts with glucose present in the sensing environment (e.g., the body of a mammal) and generates hydrogen peroxide.

As noted above, the enzyme and the second protein (e.g., an albumin) are typically treated to form a crosslinked matrix (e.g., by adding a cross-linking agent to the protein mixture). As is known in the art, crosslinking conditions may be manipulated to modulate factors such as the retained biological activity of the enzyme, its mechanical and/or operational stability. Illustrative crosslinking procedures are described in U.S. patent application Ser. No. 10/335,506 and PCT publication WO 03/035891 which are incorporated herein by reference. For example, an amine cross-linking reagent, such as, but not limited to, glutaraldehyde, can be added to the protein mixture. The addition of a cross-linking reagent to the protein mixture creates a protein paste. The concentration of the cross-linking reagent to be added may vary according to the concentration of the protein mixture. While glutaraldehyde is an illustrative crosslinking reagent, other cross-linking reagents may also be used or may be used in place of glutaraldehyde. Other suitable cross-linkers also may be used, as will be evident to those skilled in the art.

As noted above, in some embodiments of the invention, the analyte sensing constituent includes an agent (e.g., glucose oxidase) capable of producing a signal (e.g., a change in oxygen and/or hydrogen peroxide concentrations) that can be sensed by the electrically conductive elements (e.g. electrodes which sense changes in oxygen and/or hydrogen peroxide concentrations). However, other useful analyte sensing constituents can be formed from any composition that is capable of producing a detectable signal that can be sensed by the electrically conductive elements after interacting with a target analyte whose presence is to be detected. In some embodiments, the composition comprises an enzyme that modulates hydrogen peroxide concentrations upon reaction with an analyte to be sensed. Alternatively, the composition comprises an enzyme that modulates oxygen concentrations upon reaction with an analyte to be sensed. In this context, a wide variety of enzymes that either use or produce hydrogen peroxide and/or oxygen in a reaction with a physiological analyte are known in the art and these enzymes can be readily incorporated into the analyte sensing constituent composition. A variety of other enzymes known in the art can produce and/or utilize compounds whose modulation can be detected by electrically conductive elements such as the electrodes that are incorporated into the sensor designs described herein. Such enzymes include for example, enzymes specifically described in Table 1, pages 15-29 and/or Table 18, pages 111-112 of Protein Immobilization: Fundamentals and Applications (Bioprocess Technology, Vol 14) by Richard F. Taylor (Editor) Publisher: Marcel Dekker; Jan. 7, 1991) the entire contents of which are incorporated herein by reference.

### Protein Constituent

The electrochemical sensors of the invention optionally include a protein constituent disposed between the analyte sensing constituent and the analyte modulating constituent (see, e.g., element 516 in Figure 2E). The term "protein constituent" is used herein according to art accepted terminology and refers to constituent containing a carrier protein or the like that is selected for compatibility with the analyte sensing constituent and/or the analyte modulating constituent. In typical embodiments, the protein constituent comprises an albumin such as human serum albumin. The HSA concentration may vary between about 0.5%-30% (w/v). Typically, the HSA concentration is about 1-10% w/v, and most typically is about 5% w/v. In alternative embodiments of the invention, collagen or BSA or other structural proteins used in these contexts can be used instead of or in addition to HSA. This constituent is typically crosslinked on the analyte sensing constituent according to art accepted protocols.

### Adhesion Promoting Constituent

The electrochemical sensors of the invention can include one or more adhesion promoting (AP) constituents (see, e.g., element 514 in Figure 2E). The term "adhesion promoting constituent" is used herein according to art accepted terminology and refers to a constituent that includes materials selected for their ability to promote adhesion between adjoining constituents in the sensor. Typically, the adhesion promoting constituent is disposed between the analyte sensing constituent and the analyte modulating constituent. Typically, the adhesion promoting constituent is disposed between the optional protein constituent and the analyte modulating constituent. The adhesion promoter constituent can be made from any one of a wide variety of materials known in the art to facilitate the bonding between such constituents and can be applied by any one of a wide variety of methods known in the art. Typically, the adhesion promoter constituent comprises a silane compound such as γ-aminopropyltrimethoxysilane.

### High-density Amine Constituent

The electrochemical sensors of the invention can include one or more high-density amine constituent layers that provide the sensors with a number of beneficial functions. Typically, the high-density amine adhesion promoting constituent is disposed between and in direct contact with the analyte sensing constituent and the analyte modulating constituent. In typical embodiments, the high-density amine layer comprises poly-1-lysine having molecular weights between 30 KDa and 300KDa (e.g., between 150 KDa and 300KDa). The concentrations of poly-1-lysine in such high-density amine layers is typically from 0.1 weight-to-weight percent to 0.5 weight-to-weight percent and the high-density amine layer is from 0.1 to 0.4 microns thick. In embodiments where the analyte sensing layer comprises glucose oxidase so that the analyte sensor senses glucose, and the high-density amine layer functions to decrease sensor signal changes that result from fluctuating levels of oxygen (O₂).

### Analyte Modulating Constituent

The electrochemical sensors of the invention can include an analyte modulating constituent disposed on the sensor, for example an analyte modulating layer formed from a HDMSO composition. The term "analyte modulating constituent" is used herein according to art accepted terminology and refers to a constituent that typically forms a membrane on the sensor that operates to modulate the diffusion of one or more compounds, such as glucose or NAD or 3-hydroxybutyrate, through the layer/constituent. In certain embodiments of the invention, the analyte modulating constituent is an analyte-limiting membrane which operates to prevent or restrict the diffusion of one or more analytes, such as glucose, through the constituents. In other embodiments of the invention, the analyte-modulating constituent operates to facilitate the diffusion of one or more analytes, through the constituents. Optionally such analyte modulating constituents can be formed to prevent or restrict the diffusion of one type of molecule through the constituent (e.g. glucose), while at the same time allowing or even facilitating the diffusion of other types of molecules through the constituent (e.g. O₂).

FIG. 2E illustrates a cross-section of one sensor embodiment 500 of the present invention. This sensor embodiment is formed from a plurality of components that are in the form of layers of various conductive and non-conductive constituents disposed on each other according to art accepted methods and/or the specific methods of the invention disclosed herein. The components of the sensor are typically characterized herein as layers because, for example, it allows for a facile characterization of the sensor structure shown in FIG. 2E. Artisans will understand however, that in certain embodiments of the invention, the sensor constituents are combined such that multiple constituents form one or more heterogeneous layers. In this context, those of skill in the art understand that the ordering of the layered constituents can be altered in various embodiments of the invention.

The embodiment shown in Figure 2E includes a base layer 502 to support the sensor 500. The base layer 502 can be made of a material such as a metal and/or a ceramic and/or a polymeric substrate, which may be self-supporting or further supported by another material as is known in the art. Embodiments of the invention include a conductive layer 504 which is disposed on and/or combined with the base layer 502. Typically the conductive layer 504 comprises one or more electrodes. An operating sensor 500 typically includes a plurality of electrodes such as a working electrode, a counter electrode and a reference electrode. Other embodiments may also include a plurality of working, counter and reference electrodes sets that are grouped together as units.

As discussed in detail below, the base layer 502 and/or conductive layer 504 can be generated using many known techniques and materials. In certain embodiments of the invention, the electrical circuit of the sensor is defined by etching the disposed conductive layer 504 into a desired pattern of conductive paths. A typical electrical circuit for the sensor 500 comprises two or more adjacent conductive paths with regions at a proximal end to form contact pads and regions at a distal end to form sensor electrodes. An electrically insulating cover layer 506 such as a polymer coating can be disposed on portions of the sensor 500. Acceptable polymer coatings for use as the insulating protective cover layer 506 can include, but are not limited to, non-toxic biocompatible polymers such as silicone compounds, polyimides, biocompatible solder masks, epoxy acrylate copolymers, or the like. In the sensors of the present invention, one or more exposed regions or apertures 508 can be made through the cover layer 506 to open the conductive layer 504 to the external environment and to, for example, allow an analyte such as glucose to permeate the layers of the sensor and be sensed by the sensing elements. Apertures 508 can be formed by a number of techniques, including laser ablation, tape masking, chemical milling or etching or photolithographic development or the like. In certain embodiments of the invention, during manufacture, a secondary photoresist can also be applied to the protective layer 506 to define the regions of the protective layer to be removed to form the aperture(s) 508. The exposed electrodes and/or contact pads can also undergo secondary processing (e.g. through the apertures 508), such as additional plating processing, to prepare the surfaces and/or strengthen the conductive regions.

In the sensor configuration shown in Figure 2E, an analyte sensing layer 510 is disposed on one or more of the exposed electrodes of the conductive layer 504. Typically, the analyte sensing layer 510 comprises an enzyme capable of producing and/or utilizing oxygen and/or hydrogen peroxide, for example the enzyme glucose oxidase. Optionally the enzyme in the analyte sensing layer is combined with a second carrier protein such as human serum albumin, bovine serum albumin or the like. In an illustrative embodiment, an enzyme such as glucose oxidase or 3-hydroxybutyrate dehydrogenase in the analyte sensing layer 510 reacts with glucose to produce hydrogen peroxide, a compound which then modulates a current at an electrode. As this modulation of current depends on the concentration of hydrogen peroxide, and the concentration of hydrogen peroxide correlates to the concentration of glucose, the concentration of glucose can be determined by monitoring this modulation in the current. Such modulations in the current caused by changing hydrogen peroxide concentrations can by monitored by any one of a variety of sensor detector apparatuses such as a universal sensor amperometric biosensor detector or one of the other variety of similar devices known in the art such as glucose monitoring devices produced by Medtronic MiniMed. Typical sensor embodiments of this element of the invention utilize an enzyme (e.g. glucose oxidase) that has been combined with a second protein (e.g. albumin) in a fixed ratio (e.g. one that is typically optimized for glucose oxidase stabilizing properties) and then applied on the surface of an electrode to form a thin enzyme constituent. In a typical embodiment, the analyte sensing constituent comprises a GOx and HSA mixture. The enzyme and the second protein (e.g., an albumin) are typically treated to form a crosslinked matrix (e.g. by adding a cross-linking agent to the protein mixture). As is known in the art, crosslinking conditions may be manipulated to modulate factors such as the retained biological activity of the enzyme, its mechanical and/or operational stability. Illustrative crosslinking procedures are described in U.S. Patent Application Serial Number 10/335,506 and PCT publication WO 03/035891 which are incorporated herein by reference. For example, an amine cross-linking reagent, such as, but not limited to, glutaraldehyde, can be added to the protein mixture.

In embodiments of the invention, the analyte sensing layer 510 can be applied over portions of the conductive layer or over the entire region of the conductive layer. Typically, the analyte sensing layer 510 is disposed on the working electrode which can be the anode or the cathode. Optionally, the analyte sensing layer 510 is also disposed on a counter and/or reference electrode. While the analyte sensing layer 510 can be up to about 1000 microns (µm) in thickness, typically the analyte sensing layer is relatively thin as compared to those found in sensors previously described in the art, and is for example, typically less than 1, 0.5, 0.25 or 0.1 microns in thickness. As discussed in detail below, some methods for generating a thin analyte sensing layer 510 include brushing the layer onto a substrate (e.g., the reactive surface of a platinum black electrode), as well as spin coating processes, dip and dry processes, low shear spraying processes, ink-jet printing processes, silk screen processes and the like.

Typically, the analyte sensing layer 510 is coated and or disposed next to one or more additional layers. Optionally, the one or more additional layers includes a protein layer 516 disposed upon the analyte sensing layer 510. Typically, the protein layer 516 comprises a protein such as human serum albumin, bovine serum albumin or the like. Typically, the protein layer 516 comprises human serum albumin. In some embodiments of the invention, an additional layer includes an analyte modulating layer 512 that is disposed above the analyte sensing layer 510 to regulate analyte access with the analyte sensing layer 510. For example, the analyte modulating membrane layer 512 can comprise a glucose limiting membrane ("GLM"), which regulates the amount of glucose that contacts an enzyme such as glucose oxidase that is present in the analyte sensing layer.

In embodiments of the invention, an adhesion promoter layer 514 is disposed between layers such as the analyte modulating layer 512 and the analyte sensing layer 510 as shown in FIG. 2E in order to facilitate their contact and/or adhesion. In a specific embodiment of the invention, an adhesion promoter layer 514 is disposed between the analyte modulating layer 512 and the protein layer 516 as shown in FIG. 2E in order to facilitate their contact and/or adhesion. The adhesion promoter layer 514 can be made from any one of a wide variety of materials known in the art to facilitate the bonding between such layers. Embodiments of typical elements that can be included in and/or adapted for use with the sensors disclosed herein are disclosed in U.S. Patent Application Publication Nos. 20070163894, 20070227907, 20100025238, 20110319734 and 20110152654, the contents of each of which are incorporated herein by reference. For example, FIG. 2 in U.S. Patent Application Publication No. 20070163894 provides a perspective view illustrating a subcutaneous sensor insertion set, a telemetered characteristic monitor transmitter device, and a data receiving device of the type that can be adapted for use with embodiments of the invention. In addition, number of articles, U.S. patents and patent application describe the state of the art with the common methods and materials disclosed herein and further describe various elements (and methods for their manufacture) that can be used in the sensor designs disclosed herein. These include for example, U.S. Patent Nos. 6,413,393; 6,368,274; 5,786,439; 5,777,060; 5,391,250; 5,390,671; 5,165,407, 4,890,620, 5,390,671, 5,390,691, 5,391,250, 5,482,473, 5,299,571, 5,568,806; United States Patent Application 20140012115; as well as PCT International Publication Numbers WO 01/58348, WO 03/034902, WO 03/035117, WO 03/035891, WO 03/023388, WO 03/022128, WO 03/022352, WO 03/023708, WO 03/036255, WO03/036310 and WO 03/074107, the contents of each of which are incorporated herein by reference.

Embodiments of the invention include subcutaneous sensor insertion systems comprising sensors having the plasma deposited HDMSO layers as disclosed herein. FIG. 4 provides a perspective view of one generalized embodiment of subcutaneous sensor insertion system and a block diagram of a sensor electronics device according to one illustrative embodiment of the invention. Additional elements typically used with such sensor system embodiments are disclosed for example in U.S. Patent Application No. 20070163894, the contents of which are incorporated by reference. FIG. 4 provides a perspective view of a telemetered characteristic monitor system 1, including a subcutaneous sensor set 10 provided for subcutaneous placement of an active portion of a flexible sensor 12, or the like, at a selected site in the body of a user. The subcutaneous or percutaneous portion of the sensor set 10 includes a hollow, slotted insertion needle 14 having a sharpened tip 44, and a cannula 16. Inside the cannula 16 is a sensing portion 18 of the sensor 12 to expose one or more sensor electrodes 20 to the user's bodily fluids through a window 22 formed in the cannula 16. The sensing portion 18 is joined to a connection portion 24 that terminates in conductive contact pads, or the like, which are also exposed through one of the insulative layers. The connection portion 24 and the contact pads are generally adapted for a direct wired electrical connection to a suitable monitor 200 coupled to a display 214 for monitoring a user's condition in response to signals derived from the sensor electrodes 20. The connection portion 24 may be conveniently connected electrically to the monitor 200 or a characteristic monitor transmitter 100 by a connector block 28 (or the like) as shown and described in U.S. Pat. No. 5,482,473, entitled FLEX CIRCUIT CONNECTOR, which is incorporated by reference.

As shown in FIG. 4, in accordance with embodiments of the present invention, subcutaneous sensor set 10 may be configured or formed to work with either a wired or a wireless characteristic monitor system. The proximal part of the sensor 12 is mounted in a mounting base 30 adapted for placement onto the skin of a user. The mounting base 30 can be a pad having an underside surface coated with a suitable pressure sensitive adhesive layer 32, with a peel-off paper strip 34 normally provided to cover and protect the adhesive layer 32, until the sensor set 10 is ready for use. The mounting base 30 includes upper and lower layers 36 and 38, with the connection portion 24 of the flexible sensor 12 being sandwiched between the layers 36 and 38. The connection portion 24 has a forward section joined to the active sensing portion 18 of the sensor 12, which is folded angularly to extend downwardly through a bore 40 formed in the lower base layer 38. Optionally, the adhesive layer 32 (or another portion of the apparatus in contact with *in vivo* tissue) includes an anti-inflammatory agent to reduce an inflammatory response and/or antibacterial agent to reduce the chance of infection. The insertion needle 14 is adapted for slide-fit reception through a needle port 42 formed in the upper base layer 36 and through the lower bore 40 in the lower base layer 38. After insertion, the insertion needle 14 is withdrawn to leave the cannula 16 with the sensing portion 18 and the sensor electrodes 20 in place at the selected insertion site. In this embodiment, the telemetered characteristic monitor transmitter 100 is coupled to a sensor set 10 by a cable 102 through a connector 104 that is electrically coupled to the connector block 28 of the connector portion 24 of the sensor set 10.

In the embodiment shown in FIG. 4, the telemetered characteristic monitor 100 includes a housing 106 that supports a printed circuit board 108, batteries 110, antenna 112, and the cable 102 with the connector 104. In some embodiments, the housing 106 is formed from an upper case 114 and a lower case 116 that are sealed with an ultrasonic weld to form a waterproof (or resistant) seal to permit cleaning by immersion (or swabbing) with water, cleaners, alcohol or the like. In some embodiments, the upper and lower case 114 and 116 are formed from a medical grade plastic. However, in alternative embodiments, the upper case 114 and lower case 116 may be connected together by other methods, such as snap fits, sealing rings, RTV (silicone sealant) and bonded together, or the like, or formed from other materials, such as metal, composites, ceramics, or the like. In other embodiments, the separate case can be eliminated and the assembly is simply potted in epoxy or other moldable materials that is compatible with the electronics and reasonably moisture resistant. As shown, the lower case 116 may have an underside surface coated with a suitable pressure sensitive adhesive layer 118, with a peel-off paper strip 120 normally provided to cover and protect the adhesive layer 118, until the sensor set telemetered characteristic monitor transmitter 100 is ready for use.

In the illustrative embodiment shown in FIG. 4, the subcutaneous sensor set 10 facilitates accurate placement of a flexible thin film electrochemical sensor 12 of the type used for monitoring specific blood parameters representative of a user's condition. The sensor 12 monitors glucose levels in the body and may be used in conjunction with automated or semi-automated medication infusion pumps of the external or implantable type as described in U.S. Pat. No. 4,562,751; 4,678,408; 4,685,903 or 4,573,994, to control delivery of insulin to a diabetic patient.

In the illustrative embodiment shown in FIG. 4, the sensor electrodes 10 may be used in a variety of sensing applications and may be configured in a variety of ways. For example, the sensor electrodes 10 may be used in physiological parameter sensing applications in which some type of biomolecule is used as a catalytic agent. For example, the sensor electrodes 10 may be used in a glucose and oxygen sensor having a glucose oxidase enzyme catalyzing a reaction with the sensor electrodes 20. The sensor electrodes 10, along with a biomolecule or some other catalytic agent, may be placed in a human body in a vascular or non-vascular environment. For example, the sensor electrodes 20 and biomolecule may be placed in a vein and be subjected to a blood stream or may be placed in a subcutaneous or peritoneal region of the human body.

In the embodiment of the invention shown in FIG. 4, the monitor of sensor signals 200 may also be referred to as a sensor electronics device 200. The monitor 200 may include a power source, a sensor interface, processing electronics (i.e., a processor), and data formatting electronics. The monitor 200 may be coupled to the sensor set 10 by a cable 102 through a connector that is electrically coupled to the connector block 28 of the connection portion 24. In an alternative embodiment, the cable may be omitted. In this embodiment of the invention, the monitor 200 may include an appropriate connector for direct connection to the connection portion 104 of the sensor set 10. The sensor set 10 may be modified to have the connector portion 104 positioned at a different location, e.g., on top of the sensor set to facilitate placement of the monitor 200 over the sensor set.

Another embodiment of the invention disclosed herein is a method of making a sensor apparatus for implantation within a mammal from a plurality of layered elements including HMDSO layers formed using plasma deposition processes. This method can comprise the steps of: providing a base layer; forming a conductive layer on the base layer, wherein the conductive layer includes an electrode (and typically a working electrode, a reference electrode and a counter electrode); forming an analyte sensing layer on the conductive layer, wherein the analyte sensing layer includes a composition that can alter the electrical current at the electrode in the conductive layer in the presence of an analyte (as does glucose oxidase in the presence of glucose); and then forming the HMDSO layer on the analyte sensing layer.

The compositions that make up the analyte modulating layer are selected to provide a number of desirable characteristics such as contributing to sensor stability in combination with an ability to be deposited over one or more layers of the sensor using gas plasma process. Typically, such Plasma deposition processes can use a commercially available systems such a PVA-TePla^{™} M4L chamber (see, also the exemplary methods disclosed in U.S. Patent Publication Nos. 20050079200 20090081384). One general plasma deposition process includes the steps illustrated in the schematic shown in Figure 3. One illustrative step-wise procedure for use with amperometric glucose and/or ketone sensors is as follows:
1. Prepare the desired underlying layers on which the HDMSO layer is to be deposited (e.g. an analyte sensing layer comprising GOx). Pre-treat this sensor stack (e.g. a layer comprising an enzyme such as GOx) with short time gas plasma (e.g., Helium plasma, 02 plasma, or continuous wave monomer plasma) to activate the surface of the substrate.
2. Create very thin film on top of the specific layer on which the HDMSO layer is to be deposited (e.g. an analyte sensing layer comprising GOx) using HMDSO. In certain embodiments, the layer comprises 100% HMDSO and is formed using in a plasma process.

HMDSO precursors can provide siloxane groups and amino functional groups that some conventional adhesion promoter (3-aminopropyltriethoxysilane) can also provide, but don't have problematical issues associated with some conventional materials, such as low vapor pressure and high sensitivity to moisture in the air during sensor fabrication. Instead of liquid phases of the HDMSO monomers, the vapor phases are used to produce a unique plasma composition resulting in a layer comprising unique surface properties. During the pulse plasma deposition process, the HMDSO monomer vapors undergo fragmentation and reacts with the substrate and also with themselves to combine into pinhole-free films. In addition, HMDSO pulse plasma deposition generates silica like thin film layer to bind to the activated substrate, which can be a good barrier to secure an analyte modulating layer on top of an enzyme (e.g., GOx) layer underneath and to further limit analyte (e.g., glucose) permeation from an analyte modulating layer (e.g. a GLM layer) on the top.

As noted above, embodiments of the invention include the steps of forming a plasma deposited HMDSO layer. Optionally, the HMDSO layer is the analyte modulating layer. The method can also include and forming a cover layer disposed on at least a portion of the analyte modulating layer, wherein the cover layer further includes an aperture over at least a portion of the analyte modulating layer.

As discussed in detail below, the various layers of the sensor can be manufactured to exhibit a variety of different characteristics which can be manipulated according to the specific design of the sensor. Typically, a method of making the sensor includes the step of forming a protein layer on the analyte sensing layer, wherein a protein within the protein layer is an albumin selected from the group consisting of bovine serum albumin and human serum albumin. Typically, a method of making the sensor includes the step of forming an analyte sensing layer that comprises an enzyme composition selected from the group consisting of glucose oxidase and 3-hydroxybutyrate dehydrogenase. In such methods, the analyte sensing layer typically comprises a carrier protein composition in a substantially fixed ratio with the enzyme, and the enzyme and the carrier protein are distributed in a substantially uniform manner throughout the analyte sensing layer.

The disclosure provided herein includes sensors and sensor designs that can be generated using combinations of various well-known techniques. The disclosure further provides methods for applying very thin enzyme coatings to these types of sensors as well as sensors produced by such processes. In this context, some embodiments of the invention include methods for making such sensors on a substrate according to art accepted processes. In certain embodiments, the substrate comprises a rigid and flat structure suitable for use in photolithographic mask and etch processes. In this regard, the substrate typically defines an upper surface having a high degree of uniform flatness. A polished glass plate may be used to define the smooth upper surface. Alternative substrate materials include, for example, stainless steel, aluminum, and plastic materials such as delrin, etc. In other embodiments, the substrate is non-rigid and can be another layer of film or insulation that is used as a substrate, for example plastics such as polyimides and the like.

An initial step in the methods of the invention typically includes the formation of a base layer of the sensor. The base layer can be disposed on the substrate by any desired means, for example by controlled spin coating. In addition, an adhesive may be used if there is not sufficient adhesion between the substrate layer and the base layer. A base layer of insulative material is formed on the substrate, typically by applying the base layer material onto the substrate in liquid form and thereafter spinning the substrate to yield the base layer of thin, substantially uniform thickness. These steps are repeated to build up the base layer of sufficient thickness, followed by a sequence of photolithographic and/or chemical mask and etch steps to form the conductors discussed below. In an illustrative form, the base layer comprises a thin film sheet of insulative material, such as ceramic or polyimide substrate. The base layer can comprise an alumina substrate, a polyimide substrate, a glass sheet, controlled pore glass, or a planarized plastic liquid crystal polymer. The base layer may be derived from any material containing one or more of a variety of elements including, but not limited to, carbon, nitrogen, oxygen, silicon, sapphire, diamond, aluminum, copper, gallium, arsenic, lanthanum, neodymium, strontium, titanium, yttrium, or combinations thereof. Additionally, the substrate may be coated onto a solid support by a variety of methods well-known in the art including chemical vapor deposition, physical vapor deposition, or spin-coating with materials such as spin glasses, chalcogenides, graphite, silicon dioxide, organic synthetic polymers, and the like.

The methods of the invention further include the generation of a conductive layer having one or more sensing elements. Typically, these sensing elements are electrodes that are formed by one of the variety of methods known in the art such as photoresist, etching and rinsing to define the geometry of the active electrodes. The electrodes can then be made electrochemically active, for example by electrodeposition of Pt black for the working and counter electrode, and silver followed by silver chloride on the reference electrode. A sensor layer such as a sensor chemistry enzyme layer can then be disposed on the sensing layer by electrochemical deposition or a method other than electrochemical deposition such a spin coating, followed by vapor crosslinking, for example with a dialdehyde (glutaraldehyde) or a carbodi-imide.

Electrodes of the invention can be formed from a wide variety of materials known in the art. For example, the electrode may be made of a noble late transition metal. Metals such as gold, platinum, silver, rhodium, iridium, ruthenium, palladium, or osmium can be suitable in various embodiments of the invention. Other compositions such as carbon or mercury can also be useful in certain sensor embodiments. Of these metals, silver, gold, or platinum is typically used as a reference electrode metal. A silver electrode which is subsequently chloridized is typically used as the reference electrode. These metals can be deposited by any means known in the art, including an electroless method which may involve the deposition of a metal onto a previously metallized region when the substrate is dipped into a solution containing a metal salt and a reducing agent. The electroless method proceeds as the reducing agent donates electrons to the conductive (metallized) surface with the concomitant reduction of the metal salt at the conductive surface.

In an exemplary embodiment of the invention, the base layer is initially coated with a thin film conductive layer by electrode deposition, surface sputtering, or other suitable process step. In one embodiment this conductive layer may be provided as a plurality of thin film conductive layers, such as an initial chrome-based layer suitable for chemical adhesion to a polyimide base layer followed by subsequent formation of thin film gold-based and chrome-based layers in sequence. In alternative embodiments, other electrode layer conformations or materials can be used. The conductive layer is then covered, in accordance with conventional photolithographic techniques, with a selected photoresist coating, and a contact mask can be applied over the photoresist coating for suitable photoimaging. The contact mask typically includes one or more conductor trace patterns for appropriate exposure of the photoresist coating, followed by an etch step resulting in a plurality of conductive sensor traces remaining on the base layer. In an illustrative sensor construction designed for use as a subcutaneous glucose sensor, each sensor trace can include three parallel sensor elements corresponding with three separate electrodes such as a working electrode, a counter electrode and a reference electrode.

Portions of the conductive sensor layers are typically covered by an insulative cover layer, typically of a material such as a silicon polymer and/or a polyimide. The insulative cover layer can be applied in any desired manner. In an exemplary procedure, the insulative cover layer is applied in a liquid layer over the sensor traces, after which the substrate is spun to distribute the liquid material as a thin film overlying the sensor traces and extending beyond the marginal edges of the sensor traces in sealed contact with the base layer. This liquid material can then be subjected to one or more suitable radiation and/or chemical and/or heat curing steps as are known in the art. In alternative embodiments, the liquid material can be applied using spray techniques or any other desired means of application. Various insulative layer materials may be used such as photoimagable epoxyacrylate, with an illustrative material comprising a photoimagable polyimide available from OCG, Inc. of West Paterson, N.J., under the product number 7020.

Embodiments of the invention include one or more exterior cover constituents which are typically electrically insulating protective constituents (see, e.g., element 106 in Figure 2E). Typically, such cover constituents are disposed over at least a portion of the analyte modulating constituent. Acceptable polymer coatings for use as the insulating protective cover constituent can include, but are not limited to, non-toxic biocompatible polymers such as silicone compounds, polyimides, biocompatible solder masks, epoxy acrylate copolymers, or the like.

### EXAMPLES

### EXAMPLE 1: ILLUSTRATIVE WORKING EMBODIMENTS OF THE INVENTION

A plasma is a gas energized to a state of electrical conductivity in which a significant percentage of the atoms or molecules are ionized. In this state electrons, ions, radicals, excited neutrals, photons, and electric and magnetic fields are present. The collective properties of these components constitute the plasma phenomenon. Plasma-enhanced chemical vapor deposition is a process that uses plasma technology to deposit thin films from a gas state (vapor) to a solid state on a substrate. Working embodiments of the invention utilize plasma deposition technologies to form HMDSO layers useful in layered sensor structures, including a dual precursor plasma HDMSO material deposition process useful in implantable glucose sensor fabrication. Such processes avoid certain problems associated with wet chemistry HDMSO processes and provides an environment friendly alternative to conventional methods.

Using, for example, a TePla M4L Plasma Processing System, advanced dry plasma materials and processes have been developed and characterized as useful in sensor fabrication processes as well as having a biocompatibility profile that is compatible with *in vivo* implantation. Plasma deposition has many advantages over current wet chemistry processes, such as automatic process potential, significantly reducing process time, eliminating toxic glutaraldehyde and reducing daily chemical wastes.

| **Variable** | **Effect on Lubricity** | **Typical Range** | **Comments** |
|---|---|---|---|
| **Duration** | + | 40-400 sec | Longer durations result in more deposition onto the product |
| **Pressure** | +- | 50-200 mTorr | This pressure has been found to generally work well and is not typically changed |
| **RF Power** | + | 140-210 W | This power setting produces an acceptable amount of ionization of the gas mixture and is |
| | | | within the power output range of the generator |
| **HMDSO Flow Rate** | + | 10-20 sccm | Due to the large surface area of the chamber, considerable HMDSO flow is needed to provide acceptable deposition rate |
| **N₂O Flow Rate** | +- | 10-50 sccm | N₂O is an oxidizing agent that increases the oxygen content of the film and promotes adhesion to the polymer substrate |
| **AR Flow Rate** | | 1 sccm | Stabilizes plasma |

As disclosed herein, HDMSO layers formed by such processes exhibit a number of unexpected properties.

Further disclosed herein is the subject-matter of the following clauses:
1. A method of making an analyte sensor apparatus comprising:
   providing a base layer;
   forming a conductive layer over the base layer, wherein the conductive layer includes a working electrode;
   forming an analyte sensing layer over the conductive layer, wherein the analyte sensing layer includes a composition that can alter the electrical current at the working electrode in the conductive layer in the presence of an analyte;
   forming an analyte modulating layer over the analyte sensing layer wherein:
      the analyte modulating layer is formed from a material selected to inhibit the diffusion of glucose, 3-hydroxybutyrate and/or nicotinamide adenine dinucleotide therethrough; and
      the analyte modulating layer comprises hexamethyldisiloxane and is formed over the analyte sensing layer using a plasma vapor deposition process.
2. The method of clause 1, wherein the analyte sensing layer is formed to comprise an enzyme selected from the group consisting of glucose oxidase and 3-hydroxybutyrate dehydrogenase.
3. The method clause 1 or of any of the preceding clauses, wherein the analyte modulating layer is formed to be between 100nm and 1000nm in thickness.
4. The method of clause 1 or of any of the preceding clauses, further comprising forming one or more additional layers in the sensor comprising:
   an adhesion promoting layer;
   an interference rejection layer;
   a layer comprising nicotinamide adenine dinucleotide;
   a barrier layer that inhibits the diffusion of nicotinamide adenine dinucleotide therethrough; or
   a protein layer.
5. The method of clause 4, wherein:
   the barrier layer is formed to be between 100nm and 1000nm in thickness;
   the barrier layer is formed to comprise a port;
   the adhesion promoting layer and/or the interference rejection layer comprises hexamethyldisiloxane.
6. The method of clause 4 or 5, further comprising performing a pretreatment step on a layer, wherein the pretreatment step comprises exposure to a gas plasma.
7. The method of clause 1 or of any of the preceding clauses, wherein the plasma vapor deposition process comprises:
   a duration of between 40-400 seconds (e.g. between 20-50 seconds);
   a pressure of between 50 and 200 mTorr;
   a RF power of between 140-210 W;
   a hexamethyldisiloxane flow rate of between 10 and 20 sccm; and/or
   a N2O flow rate of between 10 and 50 sccm.
8. The method of clause 1 or of any of the preceding clauses, wherein the plasma vapor deposition process is a pulse deposition process or a dual plasma vapor deposition process.
9. The method of clause 1 or of any of the preceding clauses, wherein the analyte modulating layer consists essentially of hexamethyldisiloxane.
10. An analyte sensor apparatus comprising:
   a base layer;
   a conductive layer disposed over the base layer, wherein the conductive layer includes a working electrode;
   an analyte sensing layer disposed over the conductive layer, wherein the analyte sensing layer includes a composition that can alter the electrical current at the working electrode in the conductive layer in the presence of an analyte;
   an analyte modulating layer disposed over the analyte sensing layer wherein:
      the analyte modulating layer modulates the diffusion of glucose, 3-hydroxybutyrate or nicotinamide adenine dinucleotide therethrough; and
      the analyte modulating layer comprises hexamethyldisiloxane disposed over the analyte sensing layer using a plasma vapor deposition process.
11. The analyte sensor apparatus of clause 10, wherein the analyte sensing layer comprises an enzyme selected from the group consisting of glucose oxidase and 3-hydroxybutyrate dehydrogenase.
12. The analyte sensor apparatus of clause 10 or of any of clauses 10-11, wherein the analyte modulating layer is between 100nm and 1000nm in thickness.
13. The analyte sensor apparatus of clause 10 or of any of clauses 10-12, further comprising forming one or more additional layers in the sensor comprising:
   an adhesion promoting layer;
   a layer comprising nicotinamide adenine dinucleotide;
   a barrier layer that inhibits the diffusion of nicotinamide adenine dinucleotide therethrough;
   a high density amine layer; or
   a protein layer.
14. The analyte sensor apparatus of clause 13, wherein:
   the barrier layer is formed to be between 100nm and 1000nm in thickness; and/or
   the barrier layer is formed to comprise a port.
15. The analyte sensor apparatus of clause 13 or 14, wherein the adhesion promoting layer is formed so as to be in direct contact with materials in the protein layer on a first side and in direct contact with materials in the analyte modulating layer on a second side.
16. The analyte sensor apparatus of clause 10 or of any of clauses 10-15, wherein the analyte sensing layer comprises an enzyme consisting essentially of 3-hydroxybutyrate dehydrogenase.
17. The analyte sensor apparatus of clause 10 or of any of clauses 10-15, wherein the analyte sensor apparatus senses glucose.
18. The analyte sensor apparatus of clause 10 or of any of clauses 10-15, wherein the analyte sensor apparatus senses 3-hydroxybutyrate.
19. The amperometric analyte sensor of clause 13 or of any of clauses 13-18, wherein the analyte sensing layer, the interference rejection layer or the analyte modulating layer comprises:
   a diisocyanate;
   a hydrophilic polymer comprising a hydrophilic diol or hydrophilic diamine;
   a siloxane having an amino, hydroxyl or carboxylic acid functional group at a terminus; or
   a polycarbonate diol.
20. A method of sensing an analyte within the body of a mammal, the method comprising:
   implanting an electrochemical analyte sensor of clause 10 or of any of clauses 10-19 into the mammal;
   sensing an alteration in current at the working electrode in the presence of the analyte; and correlating the alteration in current with the presence of the analyte, so that the analyte is sensed.

## Claims

1. A method of making an analyte sensor apparatus comprising:
providing a base layer;
forming a conductive layer over the base layer, wherein the conductive layer includes a working electrode;
forming an analyte sensing layer over the conductive layer, wherein the analyte sensing layer includes a composition that can alter the electrical current at the working electrode in the conductive layer in the presence of an analyte;
forming an analyte modulating layer over the analyte sensing layer wherein:
the analyte modulating layer is formed from a material selected to inhibit the diffusion of glucose, 3-hydroxybutyrate and/or nicotinamide adenine dinucleotide therethrough; and
the analyte modulating layer comprises hexamethyldisiloxane and is formed over the analyte sensing layer using a plasma vapor deposition process.

2. The method of claim 1, wherein the analyte sensing layer is formed to comprise an enzyme selected from the group consisting of glucose oxidase and 3-hydroxybutyrate dehydrogenase, and/or wherein the analyte modulating layer is formed to be between 100nm and 1000nm in thickness.

3. The method of any of the preceding claims, further comprising forming one or more additional layers in the sensor comprising:
an adhesion promoting layer;
an interference rejection layer;
a layer comprising nicotinamide adenine dinucleotide;
a barrier layer that inhibits the diffusion of nicotinamide adenine dinucleotide therethrough; or
a protein layer.

4. The method of claim 3, wherein:
the barrier layer is formed to be between 100nm and 1000nm in thickness;
the barrier layer is formed to comprise a port;
the adhesion promoting layer and/or the interference rejection layer comprises hexamethyldisiloxane.

5. The method of claim 3 or 4, further comprising performing a pretreatment step on a layer, wherein the pretreatment step comprises exposure to a gas plasma.

6. The method of any of the preceding claims, wherein the plasma vapor deposition process comprises:
a duration of between 40-400 seconds (e.g. between 20-50 seconds);
a pressure of between 50 and 200 mTorr;
a RF power of between 140-210 W;
a hexamethyldisiloxane flow rate of between 10 and 20 sccm; and/or
a N2O flow rate of between 10 and 50 sccm.

7. The method of any of the preceding claims, wherein the plasma vapor deposition process is a pulse deposition process or a dual plasma vapor deposition process, and/or wherein the analyte modulating layer consists essentially of hexamethyldisiloxane.

8. An analyte sensor apparatus comprising:
a base layer;
a conductive layer disposed over the base layer, wherein the conductive layer includes a working electrode;
an analyte sensing layer disposed over the conductive layer, wherein the analyte sensing layer includes a composition that can alter the electrical current at the working electrode in the conductive layer in the presence of an analyte;
an analyte modulating layer disposed over the analyte sensing layer wherein:
the analyte modulating layer modulates the diffusion of glucose, 3-hydroxybutyrate or nicotinamide adenine dinucleotide therethrough; and
the analyte modulating layer comprises hexamethyldisiloxane disposed over the analyte sensing layer using a plasma vapor deposition process.

9. The analyte sensor apparatus of claim 8, wherein the analyte sensing layer comprises an enzyme selected from the group consisting of glucose oxidase and 3-hydroxybutyrate dehydrogenase, and/or wherein the analyte modulating layer is between 100nm and 1000nm in thickness.

10. The analyte sensor apparatus of any of claims 8 and 9, further comprising forming one or more additional layers in the sensor comprising:
an adhesion promoting layer;
a layer comprising nicotinamide adenine dinucleotide;
a barrier layer that inhibits the diffusion of nicotinamide adenine dinucleotide therethrough;
a high density amine layer; or
a protein layer.

11. The analyte sensor apparatus of claim 10, wherein:
the barrier layer is formed to be between 100nm and 1000nm in thickness; and/or
the barrier layer is formed to comprise a port.

12. The analyte sensor apparatus of claim 10 or 11, wherein the adhesion promoting layer is formed so as to be in direct contact with materials in the protein layer on a first side and in direct contact with materials in the analyte modulating layer on a second side.

13. The analyte sensor apparatus of any of claims 8-12, wherein the analyte sensing layer comprises an enzyme consisting essentially of 3-hydroxybutyrate dehydrogenase, or wherein the analyte sensor apparatus senses glucose, or wherein the analyte sensor apparatus senses 3-hydroxybutyrate.

14. The amperometric analyte sensor of any of claims 10-13, wherein the analyte sensing layer, the interference rejection layer or the analyte modulating layer comprises:
a diisocyanate;
a hydrophilic polymer comprising a hydrophilic diol or hydrophilic diamine;
a siloxane having an amino, hydroxyl or carboxylic acid functional group at a terminus; or
a polycarbonate diol.

15. A method of sensing an analyte within the body of a mammal, the method comprising:
implanting an electrochemical analyte sensor of any of claims 8-14 into the mammal;
sensing an alteration in current at the working electrode in the presence of the analyte; and correlating the alteration in current with the presence of the analyte, so that the analyte is sensed.
